# EUROPEAN PATENT SPECIFICATION

(11) **EP 4 375 367 B1**
(45) Date of publication and mention of the grant of the patent: **21.01.2026**
(21) Application number: 22860539.0
(22) Date of filing: 24.08.2022
(51) Int. Cl.: C12N 5/10, A61K 47/64, C12N 15/62, C07K 19/00, A61K 38/18, A61K 48/00, C07K 14/475, C07K 14/48, C12N 15/85, C12N 15/88

(54) **RECOMBINANT PROTEIN OF NERVE GROWTH FACTOR MUTANT AND USE THEREOF**
REKOMBINANTES PROTEIN AUS EINER NERVENWACHSTUMSFAKTORMUTANTE UND VERWENDUNG DAVON
PROTÉINE RECOMBINÉE D'UN MUTANT DU FACTEUR DE CROISSANCE NERVEUX ET SON UTILISATION

(30) Priority: 25.08.2021 CN 202110981721
(43) Date of publication of application: 29.05.2024
(73) Proprietor: Shanghai Jiao Tong University School of Medicine, Shanghai 200025 (CN); Shanghai Rnacure Biopharma Co., Ltd., Shanghai 201906 (CN)
(72) Inventor: XU, Yingjie, Huangpu District, Shanghai 20025 (CN); YU, Xiang, Huangpu District, Shanghai 20025 (CN); XU, Wei, Huangpu District, Shanghai 20025 (CN); YANG, Wen, Huangpu District, Shanghai 20025 (CN); YANG, Zheng, Huangpu District, Shanghai 20025 (CN); ZHANG, Yu, Huangpu District, Shanghai 20025 (CN)
(74) Representative: Nederlandsch Octrooibureau
(86) International application number: PCT/CN2022/114516
(87) International publication number: WO 2023/025193

(56) References cited:
- WO-A1-2010/072684
- WO-A2-2009/114702
- WO-A2-2012/170452
- CN-A- 106 008 722
- CN-A- 108 314 723
- CN-A- 110 809 476
- CN-A- 110 869 386
- CN-A- 114 933 657
- US-A1- 2008 286 323
- J. KE ET AL: "Structure and function of Norrin in assembly and activation of a Frizzled 4-Lrp5/6 complex", GENES & DEVELOPMENT, vol. 27, no. 21, 1 November 2013 (2013-11-01), US, pages 2305 - 2319, XP055417426, ISSN: 0890-9369, DOI: 10.1101/gad.228544.113
- WANG, QIAN; WANG, QIAN; WANG, ZHI-PING; XU, QIN; BAO, NAN: "Effects of Ganglioside 1 and Nerve Growth Factor on the Proliferation of Neural Stem Cells In Vitro", ZHONGGUO DANGDAI ERKE ZAZHI - CHINESE JOURNAL OF CONTEMPORARYPEDIATRICS, ZHONGNAN DAXUE,, CN, vol. 11, no. 10, 11 December 2009 (2009-12-11), CN , pages 841 - 845, XP009543863, ISSN: 1008-8830
- YANG WANLIN, SUNG KIJUNG, ZHOU FENGLI, XU WEI, RISSMAN ROBERT A., DING JIANQING, WU CHENGBIAO: "Targeted Mutation (R100W) of the Gene Encoding NGF Leads to Deficits in the Peripheral Sensory Nervous System", FRONTIERS IN AGING NEUROSCIENCE, vol. 10, XP093039573, DOI: 10.3389/fnagi.2018.00373

## Description

### TECHNICAL FIELD

The present application relates to the field of biological medicines, and particularly relates to an mRNA encoding a recombinant protein of a nerve growth factor mutant, as well as a use thereof in treatment of nervous system diseases.

### BACKGROUND

A nerve growth factor (NGF) is a growth regulatory factor with a neuronal nutritional function, playing an important role in maintaining the development and functional differentiation of central basal forebrain cholinergic neurons (BFCNs) and peripheral nociceptive sensory neurons. It is also crucial for maintaining the normal functions of mature central and peripheral neurons, as well as for self-protection and repair of nerve injuries. The NGF, as a powerful neurotrophic factor, has become a hot topic in drug therapy for various central and peripheral nervous system diseases in the past 20-30 years.

In the process of clinical research, the application of the NGF has led to dose-dependent pain side effects. Alzheimer's disease (AD) is a progressive and fatal central nervous system degenerative disease characterized by cognitive impairment and memory impairment. In clinical experiments, high-dose NGFs are used to treat AD patients, and it is found that although the cognitive ability and cortical metabolism of the AD patients are significantly improved, the occurrence of adverse reactions such as pain and weight loss offsets their therapeutic effect, ultimately declaring the failure of the clinical experiments. The same phenomenon is also found in the treatment of diabetic neuropathy and HIV peripheral neuropathy with the NGF. This is because the NGF is one of the important mediators that cause pain and hyperalgesia. Both TrkA and p75NTR signaling pathways mediate the pain inducing function of the NGF, and many studies have shown that the TrkA and p75NTR signaling pathways interact with each other. Therefore, losing any signaling pathway (TrkA or p75NTR) or breaking the balance between the TrkA and p75NTR signaling pathways will have an impact on the pain-inducing effect of the NGF.

The discovery of "painless" nerve growth factors has brought a great hope for the clinical application of the NGF. A hereditary sensory and autonomous neuropathy type V (HSAN V) secondary to an NGF^{R100W} is a mature NGF protein found in a northern Swedish family with arginine at the 100^{th} position being replaced with tryptophan, patients experience loss of pain and temperature perception, but their intelligence is normal, suggesting that the NGF^{R100W} may selectively lose the function of mediating pain, while retaining the neurotrophic function. The discovery of the NGF^{R100W} provides an important basis for studying the neurotrophic and pain mediated functions of the NGF, and the study of its biological characteristics and related mechanisms of action may seek new breakthroughs in the treatment of toxic/hereditary/metabolic peripheral neuropathy, nerve regeneration repair, and/or central nervous system degenerative diseases using the NGF.

However, a knock-in mouse model of the HSAN V demonstrates that mice expressing an NGF^{R100W} mutant typically completely lose pain at approximately 2 months of age and are often unable to survive into adulthood. Besides, a full-length NGF^{R100W} mutant protein can affect the cleavage of its mature body, thereby preventing the normal secretion of a mature NGF^{R100W} into the extracellular space to exercise its neurotrophic function. Therefore, there is currently no simple and efficient method to obtain the NGF^{R100W} mutant protein, greatly limiting the application of the NGF^{R100W} mutant protein in the treatment of nerve diseases.

Recombinant human NGF and its biological effects, variants thereof such as NGF-R100W, and variants thereof comprising a heterologous signal sequence, are described in US2008/286323A1, WO2012/170452, CN110809476A, CN106008722A, Wang et al. Zhongguo Dang Dai Er Ke Za Zhi 2009;11(10):841-845, Yang et al. Front Aging Neurosci. 2018;10:373, CN110869386A, and CN108314723A.

WO2009/114702 relates to human expression systems for recombinantly producing human proteins, including beta-NGF. WO2010/072684 describes Norrin comprising a murine IgK signal peptide.

### SUMMARY

Any references in this description to methods of treating a disease, are to be interpreted as references to compounds, agents or compositions (as disclosed herein) for use in those methods.

The invention is defined in the appended claims and relates to an isolated nucleic acid molecule encoding a recombinant protein as shown in SEQ ID NO: 15,
wherein the nucleic acid molecule is an RNA,
wherein the RNA contains modifications at one or more positions selected from: a 5' cap, a 5' untranslated region, an open reading frame, a 3 'untranslated region, and a poly A tail,
wherein the RNA contains at least one modified nucleotide,
wherein the modified nucleotide contained in the nucleic acid molecule contains pseudouridine-triphosphate (pseudo-UTP), and
wherein the nucleotide sequence of the RNA is shown in SEQ ID NO: 23.

The nucleic acid molecule of the present application encodes a recombinant protein, and the recombinant protein includes a nerve growth factor (NGF) protein variant and a heterologous signal peptide linked to the NGF protein variant. The recombinant protein has at least one of the following characteristics: (1) compared with a wild type NGF protein, binding of the NGF protein variant to a neurotrophin receptor p75 (p75NTR) is reduced; (2) an expression amount and/or a secretion amount are/is increased by at least 50%; (3) it has a considerable expression amount and/or secretion amount, for example, the expression amount and/or the secretion amount may reach at least 50% or above of those of the wild type NGF protein; (4) pain is reduced; (5) it has a prolonged half-life period; and (6) repair and regeneration of injured nerve cells are promoted. The present application further provides a composition including an mRNA encoding an NGF protein or the NGF protein variant and a delivery vector. The composition of the present application can be used for treating nervous system diseases such as peripheral neuropathy and neurodegenerative diseases and promoting nerve repair and regeneration. For example, the composition of the present application can be used for treating toxic/hereditary/metabolic peripheral neuropathy, nerve regeneration repair and/or central nervous system degenerative diseases (such as diabetic peripheral neuropathy, drug-related peripheral neuropathy, hereditary motor sensory neuropathy, post-traumatic repair of peripheral nerves, traumatic optic neuritis and/or Alzheimer's disease).

In one aspect, the recombinant protein described herein includes a nerve growth factor (NGF) protein variant and a heterologous signal peptide linked to the NGF protein variant, wherein compared with a wild type NGF protein, binding of the NGF protein variant to a neurotrophin receptor p75 (p75NTR) is reduced by at least about 50%, and
compared with a signal peptide shown in SEQ ID NO: 29, the heterologous signal peptide increases an expression amount and/or a secretion amount of the NGF protein variant by at least about 50%.

Wild type NGF protein contains an amino acid sequence shown in SEQ ID NO: 32.

According to the invention, the NGF protein variant contains an amino acid sequence shown in SEQ ID NO: 33.

According to the invention, the heterologous signal peptide is derived from mouse IgK.

According to the invention, the recombinant protein further includes a leader peptide.

According to the invention, from an N terminal to a C terminal, the recombinant protein sequentially includes the heterologous signal peptide, the leader peptide and the NGF protein variant.

The present application provides an isolated nucleic acid molecule, and the isolated nucleic acid molecule encodes the recombinant protein.

According to the invention, the isolated nucleic acid molecule is an RNA.

According to the invention, the isolated nucleic acid molecule contains modifications at one or more positions selected from: a 5' cap, a 5' untranslated region, an open reading frame, a 3' untranslated region, and a poly A tail.

According to the invention, the isolated nucleic acid molecule contains at least one modified nucleotide.

According to the invention, the modified nucleotide contained in the isolated nucleic acid molecule contains pseudouridine-5'-triphosphate (pseudo-UTP)

In another aspect, the present application provides a composition, including (a) an mRNA, containing a polynucleotide encoding a nerve growth factor (NGF) protein or a variant thereof, and (b) a delivery vector.

In certain implementations, compared with the amino acid sequence shown in SEQ ID NO: 32, binding of the NGF protein variant to a neurotrophin receptor p75 (p75NTR) is reduced by at least about 50%.

According to the invention, the NGF protein variant contains an amino acid sequence shown in SEQ ID NO: 33.

In certain implementations, the mRNA contains a polynucleotide encoding a heterologous signal peptide, and compared with a signal peptide shown in SEQ ID NO: 29, the heterologous signal peptide increases an expression amount and/or a secretion amount of the NGF protein variant by at least about 50%.

In certain implementations, the heterologous signal peptide enables the expression amount and/or the secretion amount of the NGF protein variant to reach 50% or above of those of a wild type NGF protein.

According to the invention, the heterologous signal peptide is derived from mouse IgK.

According to the invention, the mRNA further includes a polynucleotide encoding a leader peptide.

According to the invention, from the 5' terminal to the 3' terminal, the mRNA sequentially includes a polynucleotide encoding the IgK signal peptide, the polynucleotide encoding the leader peptide, and the polynucleotide encoding the NGF protein variant.

According to the invention, the mRNA contains modifications at one or more positions selected from: a 5' cap, a 5' untranslated region, an open reading frame, a 3' untranslated region, and a poly A tail.

According to the invention, the mRNA contains at least one modified nucleotide.

In certain implementations useful for understanding the invention, the modified nucleotide contained in the mRNA contains one or more nucleotides selected from: N1-methylpseudouridine-5'-triphosphate (N1-Methylpseudo-UTP), pseudouridine-5'-triphosphate (pseudo-UTP), 5-methoxyuridine-5'-triphosphate (5-Methoxy-UTP) and 5-methylcytidine-5'-triphosphate (5-Methyl-CTP). According to the invention, the modified nucleotide contained in the nucleic acid molecule contains pseudouridine-triphosphate (pseudo-UTP).

According to the invention, the mRNA contains a nucleotide sequence shown in SEQ ID NO: 23.

In certain implementations, the delivery vector includes liposome.

In certain implementations, the delivery vector includes lipid nanoparticles (LNPs).

In certain implementations, the delivery vector includes cationic lipid.

In certain implementations, a molar ratio of the cationic lipid is about 45% to about 55%.

In certain implementations, the cationic lipid is SM102 and DLin-MC3-DMA.

In certain implementations, the delivery vector includes non-cationic lipid.

In certain implementations, the non-cationic lipid includes phospholipid and/or a lipid conjugate.

In certain implementations, the phospholipid includes distearyl phosphatidylcholine (DSPC).

In certain implementations, the lipid conjugate includes a polyethylene glycol-modified lipid molecule.

In certain implementations, in the delivery vector, a molar ratio of the lipid conjugate is about 1% to about 2%.

In certain implementations, the polyethylene glycol-modified lipid molecule includes PEG2000-DMG.

In certain implementations, the delivery vector includes cholesterol.

In certain implementations, the delivery vector contains the cationic lipid, the cholesterol, the phospholipid, and the lipid conjugate, and a molar ratio of the cationic lipid to the cholesterol to the phospholipid to the lipid conjugate is 50:38.5:10:1.5.

In certain implementations, the delivery vector contains the DLin-MC3-DMA, the cholesterol, the DSPC, and PEG2000-DMG, and a molar ratio of the DLin-MC3-DMA to the cholesterol to the DSPC to the PEG2000-DMG is 50:38.5:10:1.5.

In certain implementations, the delivery vector has a diameter of about 60 nm to about 500 nm.

In certain implementations, the delivery vector has a diameter of about 80 nm to about 200 nm.

In certain implementations, the mRNA is encapsulated in the delivery vector.

In another aspect, the present application provides a vector, the vector including the nucleic acid molecule.

In another aspect, the present application provides a cell, the cell including the nucleic acid molecule and/or the vector of the present application.

In another aspect, the present application provides a method for preparing the recombinant protein, and the method includes culturing the cell under a condition of expressing the recombinant protein.

In another aspect, the present application provides a pharmaceutical composition, and the pharmaceutical composition includes the nucleic acid molecule, the composition, the vector and/or the cell, and optionally a pharmaceutically acceptable carrier.

In another aspect, the present application provides a kit or a drug administration device, and the kit or the drug administration device includes the nucleic acid molecule, the composition, the vector, the cell, and/or the pharmaceutical composition.

In another aspect, the present application provides the nucleic acid molecule, the composition, the vector, the cell and/or the pharmaceutical composition for use in relieving, preventing and/or treating central and/or peripheral nervous system diseases.

Those skilled in the art can easily gain insight into other aspects and advantages of the present application from the detailed description below. The detailed description below only shows and describes exemplary implementations of the present application. As those skilled in the art will recognize, the content of the present application enables those skilled in the art to make modifications to the disclosed specific implementations without departing from the scope of the invention which is defined by the appended claims. Correspondingly, the descriptions in the accompanying drawings and specification of the present application are only illustrative and not restrictive.

### BRIEF DESCRIPTION OF THE DRAWINGS

The specific features of the invention referred to in the present application are shown in the attached claims. By referring to the exemplary implementations and accompanying drawings described in detail below, the characteristics and advantages of the invention referred to in the present application can be better understood. A brief explanation of the accompanying drawings is as follows:
Fig. 1A shows a plurality of compositions of an NGF^{R100W} recombinant protein.
Fig. 1B shows expression situations of different NGF^{R100W} recombinant proteins in a 293T cell.
Fig. 1C shows an expression situation of an IGK19-NGFmut mRNA containing different nucleotide modifications.
Fig. 1D shows fluorescence microscope observation results of expressing and promoting PC12 cell differentiation of NGF and NGF^{R100W} recombinant protein mRNAs in a PC12 cell.
Fig. 1E shows electron microscope observation results of promoting PC12 cell differentiation of NGF and NGF^{R100W} recombinant protein mRNAs.
Fig. 1F to Fig. 1G show detection results of contents of a mature NGF protein and a mature NGF^{R100W} protein in a cell lysis solution and a cell supernatant.
Fig. 1H to Fig. 1I show detection results of contents of a mature NGF protein and a mature NGF^{R100W} protein in a cell supernatant.
Fig. 1J shows a plurality of compositions of an NGF^{R100W} recombinant protein.
Fig. 2 shows assembly and in-vivo delivery of mRNA-LNPs.
Fig. 3A shows a nano particle size distribution of NGF and NGF^{R100W} recombinant protein mRNAs.
Fig. 3B shows expression situations of NGF and NGF^{R100W} recombinant protein mRNAs in a 293T cell.
Fig. 3C shows in-vivo expression positioning of IGK19-NGFmut mRNA LNP mice.
Fig. 4A shows an impact of expression of an NGF and IGK19-NGFmut on a pain threshold of mouse heat pain.
Fig. 4B shows an impact of expression of an NGF and IGK19-NGFmut on a pain threshold of mouse mechanical pain.
Fig. 5A shows an experimental process of expression of IGK19-NGFmut on peripheral nerve injury repair in mice.
Fig. 5B shows an impact of injection of paclitaxel on pain thresholds of mouse heat pain and mechanical pain.
Fig. 5C shows a repair situation of expression of IGK19-NGFmut on injured peripheral nerves in mice.
Fig. 5D shows an impact of expression of IGK19-NGFmut on pain thresholds of heat pain and mechanical pain of mice with peripheral nerve injuries.

### DETAILED DESCRIPTION

The implementations of the present application are described below by particular specific examples, and those skilled in the art can easily understand other advantages and effects of the present application from the contents disclosed in the specification.

The technical disclosure set out below may in some respects go beyond the scope of the claims. Elements of the disclosure which do not fall within the scope of the claims are provided for information and as useful for understanding of the invention.

### Term definitions

In the present application, the term "nerve growth factor (NGF)" generally refers to a secretory protein that works in growth and survival of various neurons. As used herein, the term NGF is not limited to a human NGF, and contains all species orthologs of the human NGF. The term "NGF" covers a pro-form of the NGF, a proNGF, a full-length NGF and any form of NGF produced in an intracellular process. The term also covers NGF variants that are present naturally, such as a splicing variant, an allelic variant and an isotype.

The NGF is present in a complex of about 130-kDa composed of three proteins α-NGF, β-NGF and γ-NGF, and such NGF is also called a proNGF. The proNGF is also called an immature NGF. A γ subunit of the complex plays a role of serine protease, and can lyse an n terminal of a β subunit, thereby activating the protein to generate an NGF with functions, namely β-NGF. The NGF can bind to two receptors: a p75 neurotrophin receptor (p75NTR) and TrkA, which are transmembrane tyrosine kinases. The NGF is a fully validated target for pain, known to mediate the sensitization effect of nociceptors. An amino acid sequence of a human full-length NGF can be found in the Genbank login number NP_002497.2 or the UniProtKB login number P01138. The human full-length NGF typically contains 241 amino acids, with positions 1-18 being NGF signal peptide portions (an amino acid sequence as shown in SEQ ID NO: 29), positions 19-121 being leader peptide portions (an amino acid sequence as shown in SEQ ID NO: 34), and positions 122-241 being mature NGFs (an amino acid sequence as shown in SEQ ID NO: 32). The full-length NGF may become the mature NGF through a shearing process.

In the present application, the term "variant" generally refers to, compared to a reference sequence, an amino acid sequence that has one or more amino acids changed (such as substitution, variation, modification, replacement, deletion or addition of one or more amino acids), or a nucleic acid sequence that has one or more nucleotides changed (such as substitution, variation, modification, replacement, deletion or addition of one or more nucleotides). For polypeptides, a variant includes a polypeptide with an altered amino acid sequence due to substitution, deletion, or insertion of one or more amino acids. A variant may be present naturally or non-naturally. A variant may retain one or more activities of a reference sequence, but compared to the reference sequence, the one or more activities of the variant may be enhanced, weakened, or maintained unchanged.

In the present application, the term "NGF variant" generally refers to, compared to a full-length NGF amino acid sequence (for example, the Genbank login number NP_002497.2 or the UniProtKB login number P01138, or an amino acid sequence as shown in SEQ ID NO: 13), an amino acid sequence that has one or more amino acids changed (such as substitution, variation, modification, replacement, deletion or addition of one or more amino acids). For example, the amino acids may change in a mature NGF portion. Compared to a wild type NGF protein, the change of the amino acids (such as mutation) makes binding of an NGF protein variant to a neurotrophin receptor p75 (p75NTR) be reduced. For example, for a full-length NGF sequence with an amino acid sequence as shown in SEQ ID NO: 13, amino acid mutation may be mutation of arginine (R) at the position 221 to tryptophan (W), and the mutated full-length NGF protein may be called an NGF^{R221W} (an amino acid sequence as shown in SEQ ID NO: 14). For example, for a mature NGF sequence with an amino acid sequence as shown in SEQ ID NO: 32, amino acid mutation may be mutation of arginine (R) at the position 100 to tryptophan (W), and the mutated mature NGF protein may be called an NGF^{R100W} (an amino acid sequence as shown in SEQ ID NO: 33).

In the present application, the term "link" may be used interchangeably with "fused" or "fusion", and generally refers to the link of two or more elements or components together through any means including chemical conjugation or recombination. In the case of polypeptides, two polymers of amino acid residues are directly coupled to each other, or in the case of polynucleotides, two polymers of nucleotides are directly coupled to each other, or separated by inserting amino acid residues or nucleotides within the same polypeptide or polynucleotide.

In the present application, the term "heterologous" generally refers to a polypeptide or nucleic acid containing two or more sequences or subsequences that have not been found in the same relationship with each other in nature. For example, recombination typically produces expression boxes that have two or more sequences from unrelated genes to arrange into new functional nucleic acids. The term "heterologous signal peptide" generally refers to the fact that in natural circumstances, the signal peptide and an amino acid sequence linked to the signal peptide do not belong to the same origin, may be from different species and different individuals, may be from the same species but different individuals, may be from the same individual but different proteins, and may be from the same species but different proteins. For example, the heterologous signal peptide and the amino acid sequence linked to the heterologous signal peptide are encoded by naturally unrelated genes to arrange into new polypeptides. For example, when a signal peptide is linked to an NGF protein or an NGF protein variant linked to the signal peptide, it can be said that the signal peptide is heterologous when the signal peptide and the NGF protein or the NGF protein variant are encoded by naturally unrelated genes, that is, when the gene encoding the signal peptide is not related to the gene encoding the NGF protein or the NGF protein variant, or when the protein linked to the signal peptide is not an NGF protein or an NGF protein variant in natural circumstances.

In the present application, the term "signal peptide" generally refers to a short peptide chain that guides the transfer of newly synthesized proteins to a secretion pathway. The general length of a signal peptide is 5-30 amino acids. A signal peptide may be located at an N terminal or C terminal of a protein pro-form, but in most cases, it exists as an N-terminal peptide on the protein pro-form. The function of a signal peptide may be to promote the translocation of expressed polypeptides connected to the endoplasmic reticulum. A signal peptide is usually excised in this process. A signal peptide with a protein precursor excised may be heterologous or homologous to organisms used to produce polypeptides. For example, the signal peptide of the present application may contain functional active fragments, truncated bodies, and/or mutants of wild type signal peptides. For example, the signal peptide of the present application may have the function of guiding substances containing signal peptides to bind to cell membranes.

In the present application, the term "wild type" generally refers to a sequence of amino acids or nucleic acids that naturally exist within a species or population (such as human, mice, rats, and cells).

In the present application, the term "neurotrophin receptor p75" generally refers to a low-affinity nerve growth factor receptor (LNGFR), may bind to a neurotrophin and is a member of a tumor necrosis factor receptor superfamily, also known as a p75NTR. An amino acid sequence and other information of an exemplary human p75NTR can be found in the UniProtKB database login number P08138. As used herein, the p75NTR is not limited to a human p75NTR, and contains all species orthologs of the human p75NTR. The term "p75NTR" covers a pro-form of the p75NTR, a p75NTR precursor, a full-length p75NTR and any form of p75NTR produced in an intracellular process. The term also covers p75NTR variants that are present naturally, such as a splicing variant, an allelic variant and an isotype.

In the present application, the term "neurotrophin" generally refers to a protein that provides supports for survival, development and normal function realization of neurons. It is one of growth factors. An exemplary neurotrophin may include a nerve growth factor (NGF), a brain-derived neurotrophic factor (BDNF), a neurotrophin-3 (NT-3), a neurotrophin-4 (NT-4), a neurotrophin-6 (NT-6), a neurotrophin-7 (NT-7), dehydroepiandrosterone (DHEA), DHEA sulphate (DHEA-S), a GDNF ligand family, a ciliary neurotrophic factor (CNTF) and other biological molecules.

In the present application, the term "brain-derived neurotrophic factor (BDNF)" generally refers to one of neurotrophic factors, and may also be called "abrineurin". The BDNF is a member of a neurotrophic factor growth factor family, and is related to a classic nerve growth factor. The BDNF is typically present in the brain and the periphery. As used herein, the BDNF is not limited to a human BDNF, and contains all species orthologs of the human BDNF. The term "BDNF" covers a pro-form of the BDNF, a BDNF precursor, a full-length BDNF and any form of BDNF produced in an intracellular process. The term also covers BDNF variants that are present naturally, such as a splicing variant, an allelic variant and an isotype. An amino acid sequence of a human BDNF protein can be found in the UniProtKB database login number P23560. A full-length human BDNF protein typically has 247 amino acids, and may contain a signal peptide, a precursor part, a leader peptide and a mature BDNF. The BDNF signal peptide may be amino acids at positions 1-18 of the full-length human BDNF protein, for example, the BDNF signal peptide may contain an amino acid sequence shown in SEQ ID NO: 31.

In the present application, the term "neurotrophin-3 (NT-3)" generally refers to a neurotrophic factor in a neurotrophic factor NGF (nerve growth factor) family. The NT-3 is a protein growth factor, and has activities on certain neurons of peripheral and central nervous systems. An amino acid sequence of an exemplary human NT-3 can be found in the UniProtKB database login number P20783.

In the present application, the term "neurotrophin-4 (NT-4)" generally refers to a neurotrophic factor in a neurotrophic factor NGF (nerve growth factor) family. The NT-4 sends a signal mainly through a TrkB receptor tyrosine kinase. The NT-4 may also be called an NT-5. An amino acid sequence of an exemplary human NT-4 can be found in the UniProtKB database login number P34130.

In the present application, the term "leader peptide" generally refers to an excised part in the mature or activation process of proteins. Once excised, the leader peptide usually does not have independent biological functions. The leader peptide may assist in folding and correct expression of proteins. In some cases, the leader peptide may contain a signal peptide or a part of the signal peptide. In some cases, the leader peptide does not contain a signal peptide.

In the present application, the term "delivery vector" generally refers to a transfer medium which can deliver a reagent (such as an mRNA) to a target cell. The delivery vector may deliver the reagent (such as the mRNA) to a specific cell subclass. For example, in virtue of the inherent features of the delivery vector or through a part coupled to the vector and a part contained in the delivery vector (or a part combined with the vector to maintain the part together with the delivery vector, thereby making the part sufficient to target the delivery vector), the delivery vector is made to target certain types of cells. The delivery vector may also increase the in-vivo half-life period of the reagent to be delivered (such as the mRNA) and/or the bioavailability of the reagent to be delivered. The delivery vector may include a viral vector, viral like particles, a polycationic vector, a peptide vector, liposome, and/or a hybrid vector. For example, if a target cell is a liver cell, the property of the delivery vector (such as the size, charges, and/or pH) may effectively deliver the delivery vector and/or molecules (such as the mRNA) encapsulated therein to the target cell, reduce immune clearance, and/or promote residence in the target cell.

In the present application, the term "lipid nanoparticle (LNP)" generally refers to a particle containing a plurality of (i.e., more than one) lipid molecules that physically bind to each other (such as covalently or non-covalently) through intermolecular force. The lipid nanoparticle may be, for example, a microsphere (including unilamellar and multilamellar vesicles, such as liposome), a dispersion phase in an emulsion, a micelle or an internal phase in a suspension. The lipid nanoparticle may contain one or more lipids (such as cationic lipids, non-cationic lipids and PEG-modified lipids).

In the present application, the term "liposome" generally refers to a vesicle having an internal space and isolated from an external medium through one or more double-layer membranes. For example, the double-layer membrane may be formed from amphiphilic molecules, such as synthesized or natural lipids containing spatially-isolated hydrophilic and hydrophobic domains; and for another example, the double-layer membrane may be formed from an amphiphilic polymer and a surfactant.

In the present application, when used for a nucleic acid (such as an RNA or a DNA), the term "modification" generally refers to, compared to a corresponding wild type, the nucleic acid has different nucleotide molecules, has different nucleotide sequences, is composed of different bonds and/or is doped with non-natural parts in its structure. For example, the modification may include modification to nucleotide, for example, the nucleotide may include a modified base, saccharide or phosphate group. For example, the modification may include polypeptides or proteins with different nucleotide sequences but encoding the same amino acid sequence, or polypeptides or proteins with the same function. The modification may be chemical modification and/or biological modification. "Chemical modification" may include the introduction of chemical substances that are different from those seen in wild type or naturally present nucleic acids, such as covalent modification, such as the introduction of modified nucleotides (such as nucleotide analogues, or the introduction of side groups that are not naturally found in these nucleic acid molecules). The term "modified nucleotide" usually refers to a unit in a nucleic acid polymer, and it contains a modified base, saccharide, or phosphate group, or is doped with non-natural parts in its structure.

In the present application, when used for a nucleic acid, the term "codon-optimization" generally represents a polypeptide-encoding nucleic acid that has been improved to have improved expression in a cell by replacing one, at least one, or one or more codons in a parental polypeptide-encoding nucleic acid with codons which have different relative use frequencies in a cell and encode the same amino acid residue.

In the present application, the term "polynucleotide" generally includes a DNA molecule (such as a cDNA or a genomic DNA), an RNA molecule (such as an mRNA), a DNA or RNA anologue produced using nucleotide analogues (such as a peptide nucleic acid and a non-naturally present nucleotide analogue), and their heterozygote. A nucleic acid molecule may be single-stranded or double-stranded.

In the present application, the term "isolated nucleic acid molecule" generally refers to a single or double stranded polymer or its analogues of deoxyribonucleotide or ribonucleotide bases read from the 5' terminal to the 3' terminal, and it has been isolated from at least about 50% of polypeptides, peptides, lipids, saccharides, polynucleotides, or other materials naturally discovered together with nucleic acid molecules when isolating total nucleic acids from source cells. For example, the isolated nucleic acid molecule generally does not contain any other polluting nucleic acid molecules or other molecules found in the natural environment of nucleic acids that can interfere with its use or its therapeutic, diagnostic, preventive, or research uses.

In the present application, the term "mRNA" generally refers to an RNA transcript that has been treated to remove introns and can be translated into polypeptides.

In the present application, the term "vector" generally refers to a nucleic acid molecule that can self-replicate in suitable hosts, and it transfers an inserted nucleic acid molecule to host cells and/or between the host cells. The vector may include a vector primarily used for inserting a DNA or an RNA into a cell, a vector primarily used for replicating a DNA or an RNA, and a vector primarily used for expression of transcription and/or translation of a DNA or an RNA. The vector also includes a vector with multiple aforementioned functions. The vector may be a polynucleotide that can be transcribed and translated into polypeptides when introduced into a suitable host cell. Usually, by culturing suitable host cells containing the vector, the vector may produce a desired expression product.

In the present application, the term "cell" generally refers to an individual cell, cell line, or cell culture that may or has already contained a plasmid or vector including the nucleic acid molecule described in the present application, or that can express antibodies or their antigen-binding fragments described in the present application. The cell may include a filial generation of a single host cell. Due to natural, accidental, or intentional mutations, a daughter cell may not necessarily be identical in morphology or genome to original parent cells, but can express the antibodies or their antigen-binding fragments described in the present application. The cell may be obtained by in vitro transfection of cells using the vector described in the present application. The cell may be a prokaryotic cell or a eukaryotic cell.

In the present application, the term "pharmaceutically acceptable carrier" generally includes a pharmaceutically acceptable carrier, excipient, or stabilizer that is non-toxic to cells or mammals exposed to it at the dosage and concentration adopted. Usually, a physiologically acceptable carrier is a pH buffered aqueous solution. Examples of the physiologically acceptable carrier may include a buffering agent, an antioxidant, a low molecular weight (less than about 10 residues) polypeptide, a protein, a hydrophilic polymer, an amino acid, monosaccharides, disaccharides, and other carbohydrates, a chelating agent, sugar alcohols, and salt forming counter ions, such as sodium; and/or a non-ionic surfactant.

In the present application, the term "pharmaceutical composition" generally refers to a preparation in the form allowing the biological activity of active ingredients (such as an S-protein variant and the nucleic acid molecule of the present application) to be effective, and it does not contain other ingredients that have unacceptable toxicity to a subject to which the preparation is to be administrated. The preparation may be sterile.

In the present application, the term "kit" generally refers to a packaged product containing components used to treat PD-1 mediated related diseases by administrating antigen binding proteins of the present application. The components of the kit may be contained in separate vials (i.e., a kit with separate parts) or provided in a single vial. The kit may include reagents such as buffering agents, protein stabilizing reagents, signal generation systems (such as fluorescence signal generation systems), antibodies, control proteins, and test containers. The kit may also contain instructions for implementing the method.

In the present application, the term "drug administration device" includes: (i) an infusion module for administering a pharmaceutical composition containing an active ingredient to a subject; (ii) a pharmaceutical composition for infusion, the pharmaceutical composition containing an active ingredient selected from: a recombinant protein, a nucleic acid molecule, an mRNA, a vector, a cell, a composition, a pharmaceutical composition, or a combinations thereof; and (iii) an optional drug efficacy monitoring module.

In the present application, the term "central and/or peripheral nervous system disease" generally refers to diseases related to lesions of a central nervous system (brain or spinal cord) and a peripheral nervous system (nerves outside the brain or spinal cord). For example, the central and/or peripheral nervous system disease may manifest as being caused by one or more of the following: neuronal degeneration, inflammation or loss of oligodendrocytes, cerebral blood supply infarction, injury or tumor and bacterial or viral infections. The central and/or peripheral nervous system disease may include Alzheimer's disease, Huntington's chorea, Parkinson's disease, multiple sclerosis, encephalitis or meningitis, damage to brain or spinal cord structures, and/or stroke.

In the present application, the term "neurodegenerative disease" generally refers to the gradual loss of neuronal functions and structures leading to cognitive impairment. The neurodegenerative disease may include Alzheimer's disease, Parkinson's disease, Huntington's chorea, early-onset Alzheimer's disease or early-onset Parkinson's disease, and/or amyotrophic lateral sclerosis.

In the present application, the term "peripheral neuropathy" generally refers to a disease or condition caused by abnormalities in a peripheral nervous system. The peripheral nervous system includes all nerves except the central nervous system (brain and spinal cord), such as nerves (brain nerves) that connect the head, face, eyes, nose, muscles, and ears to the brain, nerves that connect the spinal cord to other parts of the body, and nerve cells distributed throughout various parts of the body.

In the present application, the term "subject" generally refers to human or non-human animals (including mammals) that require diagnosis, prognosis, improvement, prevention, and/or treatment of diseases, such as human, non-human primates (apes, gibbons, gorillas, chimpanzees, orangutans, and macaques), domestic animals (dogs and cats), farm animals (poultry such as chickens and ducks, horses, cows, goats, sheep, and pigs), and experimental animals (mice, rats, rabbits, and guinea pigs). Human subjects include fetuses, newborns, infants, adolescents, and adult subjects. The subject may include an animal disease model.

In the present application, the terms "include", "comprise", "have", "may", "contain" and their variants are typically intended to be open-ended transitional phrases, terms, or words, and do not exclude the possibility of additional behaviors or structures. The term "composed of" generally represents that there cannot be other components (or similarly, features, integers, steps, etc.). Unless otherwise explicitly stated in the context, singular forms such as "a", "an", and "the" in English, and "a", "an", and "said/the" in Chinese generally include the plural form of the thing referred to.

In the present application, the term "about" generally refers to approximately, in the region of, roughly, or around. When the term "about" is used to refer to a range of numerical values, a cutoff or specific value is used to indicate that the stated value may differ by up to 10% from the listed values. Therefore, the term "about" may be used to cover variations of ±10% or less, ±5% or less, ±1% or less, ±0.5% or less, or ±0.1% or less from a specific value.

### Detailed description of the present invention

### Recombinant protein

The nucleic acid molecule of the present application encodes a recombinant protein, and the recombinant protein includes a nerve growth factor (NGF) protein variant, wherein compared with a wild type NGF protein, binding of the NGF protein variant to a neurotrophin receptor p75 (p75NTR) is reduced by at least about 50% (for example, reduced by at least about 55%, at least about 60%, at least about 65%, at least about 70%, at least about 75%, at least about 80%, at least about 85%, at least about 90%, at least about 95%, at least about 98% or above), for example, the wild type NGF protein may contain an amino acid sequence shown in SEQ ID NO: 32.

In the present application, compared with the amino acid sequence shown in SEQ ID NO: 32, the NGF protein variant contains one amino acid mutation. Compared with the amino acid sequence shown in SEQ ID NO: 32, arginine at position 100 of the NGF protein variant is mutated to tryptophan (i.e., R100W). The NGF protein variant contains an amino acid sequence shown in SEQ ID NO: 33.

It has been known in the art that when a mature NGF protein has R100W mutation, its ability of being secreted out of a cell is lowered. Described herein is a recombinant protein of an NGF^{R100W} variant with an expression amount and/or secretion amount increased compared with a naturally present NGF^{R100W} variant protein. This recombinant protein can increase the amount of secretion out of a cell of a mature NGF^{R100W} variant protein. The recombinant protein contains a heterologous signal peptide, and the heterologous signal peptide is located at the N terminal of the NGF protein variant. For example, compared with a signal peptide shown in SEQ ID NO: 29, the heterologous signal peptide increases the expression amount and/or the secretion amount of the NGF protein variant by at least about 50% (for example, increased by at least about 55%, at least about 60%, at least about 65%, at least about 70%, at least about 75%, at least about 80%, at least about 85%, at least about 90%, at least about 95%, at least about 98% or above).

The heterologous signal peptide of the present application comes from mice.

The heterologous signal peptide of the present application is derived from Mus musculus. The heterologous signal peptide of the present application contains a signal peptide derived from Igκ. The heterologous signal peptide is derived from mouse IgK.

The heterologous signal peptide is an IgK signal peptide, and the heterologous signal peptide contains an amino acid sequence shown in SEQ ID NO: 30.

In the present application, the recombinant protein further includes a leader peptide. The leader peptide contains an amino acid sequence shown in SEQ ID NO: 34.

In the present application, the recombinant protein further includes a leader peptide. In the present application, from an N terminal to a C terminal, the recombinant protein sequentially includes the heterologous signal peptide, the leader peptide and the NGF protein variant.

In the present application, the recombinant protein further includes a leader peptide. In the present application, from an N terminal to a C terminal, the recombinant protein sequentially includes the heterologous signal peptide, the leader peptide and the NGF protein variant, the heterologous signal peptide contains an amino acid sequence shown in SEQ ID NO: 30, the leader peptide contains an amino acid sequence shown in SEQ ID NO: 34, and the NGF protein variant contains an amino acid sequence shown in SEQ ID NO: 33.

From the N terminal to the C terminal, the recombinant protein sequentially includes the IgK signal peptide, the leader peptide and the NGF protein variant. The recombinant protein contains an amino acid sequence shown in SEQ ID NO: 15.

The recombinant protein further includes a tag peptide.

From the N terminal to the C terminal, the recombinant protein sequentially includes the heterologous signal peptide, the leader peptide, the NGF protein variant and the tag peptide.

For example, in aspects useful for understanding the invention, the tag peptide may contain an amino acid sequence shown in (YPYDVPDYA)n, wherein n is any integer from 1 to 5. In another example useful for understanding the invention, the tag peptide may contain an amino acid sequence shown in YPYDVPDYA (SEQ ID NO: 68) or YPYDVPDYAYPYDVPDYA (SEQ ID NO: 69). According to the invention, the tag peptide contains an amino acid sequence shown in YPYDVPDYA (SEQ ID NO: 68).

From the N terminal to the C terminal, the recombinant protein sequentially includes the IgK signal peptide, the leader peptide, the NGF protein variant and the tag peptide. The recombinant protein contains an amino acid sequence shown in SEQ ID NO: 70.

### Nucleic acid molecule

In another aspect, the present application provides an isolated nucleic acid molecule, and the isolated nucleic acid molecule encodes the recombinant protein. According to the invention, the isolated nucleic acid molecule encodes a recombinant protein containing an amino acid sequence shown in SEQ ID NO: 15.

According to the invention, the isolated nucleic acid molecule is an RNA, and encodes a recombinant protein containing an amino acid sequence shown in SEQ ID NO: 15.

According to the invention, the isolated nucleic acid molecule contains modifications at one or more positions selected from: a 5' cap, a 5' untranslated region, an open reading frame, a 3' untranslated region, and a poly A tail.

According to the invention, the isolated nucleic acid molecule contains at least one modified nucleotide.

According to the invention, the nucleic acid molecule contains a nucleotide sequence shown in SEQ ID NO: 23.

In the present application, the nucleic acid molecule contains a nucleotide sequence shown in SEQ ID NO: 23.

The present application provides an isolated RNA, particularly an mRNA. The mRNA contains a nucleic acid molecule which can encode a nerve growth factor (NGF) protein variant , or, at least a part of the mRNA can encode the NGF protein variant.

In certain implementations, compared with the amino acid sequence shown in SEQ ID NO: 32, binding of the NGF protein variant to a neurotrophin receptor p75 (p75NTR) is reduced by at least 50% (for example, reduced by at least 55%, at least 60%, at least 65%, at least 70%, at least 75%, at least 80%, at least 85%, at least 90%, at least 95%, at least 98% or above).

According to the invention, the NGF protein variant contains an amino acid sequence shown in SEQ ID NO: 33.

In certain implementations, the mRNA contains a polynucleotide encoding a heterologous signal peptide, and compared with a signal peptide shown in SEQ ID NO: 29, the heterologous signal peptide increases an expression amount and/or a secretion amount of the NGF protein variant by at least 50% (for example, increased by at least 55%, at least 60%, at least 65%, at least 70%, at least 75%, at least 80%, at least 85%, at least 90%, at least 95%, at least 98% or above).

In certain implementations, the heterologous signal peptide enables the expression amount and/or the secretion amount of the NGF protein variant to reach 50% or above (for example, reach at least 55%, at least 60%, at least 65%, at least 70%, at least 75%, at least 80%, at least 85%, at least 90%, at least 95%, at least 98% or above) of those of a wild type NGF protein.

According to the invention, the heterologous signal peptide is derived from mouse IgK.

According to the invention, the heterologous signal peptide is an IgK signal peptide.

According to the invention, the heterologous signal peptide contains an amino acid sequence shown in SEQ ID NO: 30.

According to the invention, the mRNA further includes a polynucleotide encoding a leader peptide.

According to the invention, the leader peptide contains an amino acid sequence shown in SEQ ID NO: 34.

According to the invention, from the 5' terminal to the 3' terminal, the mRNA sequentially includes a polynucleotide encoding the IgK signal peptide, the polynucleotide encoding the leader peptide, and the polynucleotide encoding the NGF protein variant.

According to the invention, the mRNA encodes and contains an amino acid sequence shown in SEQ ID NO: 15.

The present application provides an isolated RNA, particularly an mRNA. The mRNA contains a nucleic acid molecule which can encode the recombinant protein, or, at least a part of the mRNA can encode the recombinant protein, wherein, the recombinant protein contains an amino acid sequence shown in SEQ ID NO: 15.

In the present application, the RNA may be a naturally or non-naturally present RNA, such as the mRNA. The mRNA may include one or more nucleic bases, nucleosides or nucleotides. In the present application, the term "nucleoside" generally refers to a compound containing sugar molecules (such as pentose or ribose) or derivatives and organic bases (such as purine and pyrimidine) thereof or a combination of derivatives (also called "nucleic bases" herein) thereof. In the present application, a "nucleotide" generally refers to a nucleoside including a phosphate group.

In the present application, the mRNA may include a 5' untranslated region (5' UTR), a 3' untranslated region (3' UTR) and/or a coding region (such as an open reading frame).

In the present application, the mRNA is modified. The modification may be chemical modification or biological modification. In the present application, the mRNA contains one or more modified nucleic bases, nucleosides or nucleotides, and at the moment, it may be called a "chemically modified mRNA", and may also be called a "modified mRNA" herein. Compared with a non-modified reference sequence (such as a naturally present or wild type mRNA), the modified mRNA may have useful properties, e.g., including enhanced stability, improved intracellular retention, an enhanced translation effect and/or lowered immunogenicity. Therefore, using the modified mRNA may improve the protein production efficiency, improve the retention of nucleic acids in cells and lower immunogenicity.

According to the invention, the mRNA contains modifications at one or more positions selected from: a 5' cap, a 5' untranslated region, an open reading frame, a 3' untranslated region, and a poly A tail.

In the present application, the mRNA may include a modified nucleic base, which may be modified uracil. In the present application, the mRNA may include a modified nucleic base, which may be modified cytosine. In the present application, the mRNA may include a modified nucleic base, which may be modified adenine. In the present application, the mRNA may include a modified nucleic base, which may be modified guanine. In the present application, the mRNA may include a combination of one or more aforementioned modified nucleic bases (such as 2, 3 or 4 combinations of the aforementioned modified nucleic bases).

For example, the isolated nucleic acid molecule may be one or more nucleotides selected from: N1-methylpseudouridine-5'-triphosphate (N1-Methylpseudo-UTP), pseudouridine-5'-triphosphate (pseudo-UTP), 5-methoxyuridine-5'-triphosphate (5-Methoxy-UTP) and 5-methylcytidine-5'-triphosphate (5-Methyl-CTP). In certain embodiments, the modified RNA of the present application includes a combination of one or more aforementioned modified nucleic bases (such as 2, 3 or 4 combinations of the aforementioned modified nucleic bases). According to the invention, the RNA contains at least one modified nucleotide, wherein the modified nucleotide contained in the nucleic acid molecule contains pseudouridine-triphosphate (pseudo-UTP).

According to the invention, the mRNA contains a nucleotide sequence shown in SEQ ID NO: 23.

The mRNA of the present application may be produced through any feasible method in the art, including but not limited to in vitro transcription (IVT) and synthesis methods. The mRNA may be prepared using enzymatic, solid-phase, liquid-phase, combinatorial synthesis methods, small domain synthesis, and ligation methods. In one embodiment, the mRNA is prepared using an IVT enzymatic synthesis method. Therefore, the present application further relates to polynucleotides, such as DNAs, constructs, and vectors, that may also be used for in vitro transcription of the mRNA in the present application.

### Composition

In the present application, the composition may further contain a delivery vector. The mRNA of the present application may be prepared in nanoparticles or other delivery vectors, for example, to avoid being degraded when delivered to a subject. In the present application, the mRNA may be encapsulated in nanoparticles. In a specific embodiment, the nanoparticles are particles with at least one size (such as a diameter) being less than or equal to about 1000 nM, less than or equal to about 500 nM, less than or equal to about 400 nM, less than or equal to about 300 nM, less than or equal to about 200 nM, or less than or equal to about 100 nM. In a specific embodiment, the nanoparticles may include lipids. Lipid nanoparticles may include, but are not limited to liposome and micelles. In the present application, the lipid nanoparticles may include cationic and/or ionizable lipids, anionic lipids, non-cationic lipids, neutral lipids, amphiphilic lipids, polyethylene glycolated lipids, and/or structural lipids, or a combination of the above. In certain embodiments, the lipid nanoparticles contain one or more RNAs, such as the mRNA, as described in the present application, for another example, an mRNA encoding a target polypeptide (such as the recombinant protein or the NGF protein variant). For example, the lipid nanoparticles contain one or more mRNAs encoding the recombinant protein or the NGF protein variant of the present application.

The delivery vector in the composition of the present application may be the lipid nanoparticles. The lipid nanoparticles may contain one or more (such as 1, 2, 3, 4, 5, 6, 7 or 8) cationic and/or ionizable lipids. The "cationic lipid" generally refers to a lipid carrying any number of net positive charges under certain pH (such as physiological pH). The cationic lipid may include, but is not limited to 3-(didodecylamino)-N1,N1,4-dotriacontyl-1-piperazine ethylamine (KL10), N1-[2-(docosylamino)ethyl]-N1,N4,N4-dotriacontyl-1,4-piperazine diethylamine (KL22), 14,25-tricosyl-15,18,21,24-tetraazaoctaporealkane (KL25), DLin-DMA, DLin-K-DMA, DLin-KC2-DMA, Octyl-CLinDMA, Octyl-CLinDMA (2S), DODAC, DOTMA, DDAB, DOTAP, DOTAP.C1, DC-Choi, DOSPA, DOGS, DODAP, DODMA and DMRIE. In addition, many commercial cationic and/or ionizable lipids may be used, such as LIPOFECTIN^{®} (including DOTMA and DOPE) and LIPOFECTAMINE^{®} (including DOSPA and DOPE). For example, the cationic lipid may be DLin-MC3-DMA or DLin-KC2-DMA.

In certain implementations, a molar ratio of the cationic lipid in the lipid nanoparticles is about 40%-70%, such as about 40%-65%, about 40%-60%, about 45%-55% or about 48%-53%. In certain implementations, a molar ratio of the cationic lipid (such as DLin-MC3-DMA) in the lipid nanoparticles is about 50%.

In the present application, the lipid nanoparticles may contain one or more (such as 1, 2, 3, 4, 5, 6, 7 or 8) non-cationic lipids. The non-cationic lipids may include anionic lipids. The anionic lipids suitable for the lipid nanoparticles of the present application may include phosphatidylglycerol, cardiolipin, diacylphosphatidylserine, diacylphosphatidic acid, N-lauroylphosphatidylethanolamine, N-succinylphosphatidylethanolamine, N-glutarylphosphatidylphosphoethanol, and other neutral lipids to which anionic groups are linked.

The non-cationic lipids may include neutral lipids. The neutral lipids suitable for the lipid nanoparticles of the present application may include phospholipid, such as distearoylphosphatidylcholine (DSPC), dioleoylphosphatidylcholine (DOPC), dipalmitoylphosphatidylcholine (DPPC), dioleoylphosphatidylglycerol (DOPG), dipalmitoylphosphatidylglycerol (DPPG), dioleoylphosphatidylethanolamine (DOPE), palmitoyloleoylphosphatidylcholine (POPC), palmitoyloleoyl- Phosphatidylethanolamine (POPE), dioleoyl-phosphatidylethanolamine 4-(N-maleimidomethyl)-cyclohexane-1-carboxylate (DOPE-mal), dipalmitoylphosphatidylethanolamine (DPPE), dimyristoylphosphoethanolamine (DMPE), distearoyl-phosphatidyl-ethanolamine (DSPE), 16-O-monomethylPE, 16-O-dimethylPE, 18-1-transPE, 1-stearoyl-2-oleoyl-phosphatidylethanolamine (SOPE), or mixtures thereof. In addition, lipids having a mixture of saturated and unsaturated fatty acid chains may be used. For example, the neutral lipids in the present application may be selected from DOPE, DSPC, DPPC, POPC or any related phosphatidylcholine.

In the present application, the lipid nanoparticles may contain a lipid conjugate, such as a polyethylene glycol (PEG) modified lipid and a derived lipid. The PEG-modified lipid may include, but is not limited to a polyethylene glycol chain up to 5 kDa in length covalently linked to a lipid having an alkyl chain of a C6-C20 length. The addition of these components may prevent lipid aggregation, increase circulation duration, facilitate delivery of lipid-nucleic acid compositions to target cells, or rapidly release nucleic acids. For example, the polyethylene glycol (PEG) modified lipid molecule may be a PEG-ceramide with a shorter acyl chain (e.g., C14 or C18). For example, the lipid nanoparticles may contain PEG2000-DMG.

In certain implementations, a molar ratio of the polyethylene glycol (PEG) modified lipid molecule in the lipid nanoparticles is about 0.5%-2%, such as about 1%-2%, about 1.2%-1.8%, or about 1.4%-1.6%. In certain implementations, a molar ratio of the polyethylene glycol (PEG) modified lipid molecule (such as PEG2000-DMG) in the lipid nanoparticles is about 1.5%.

In certain implementations, a molar ratio of the cationic lipids to the cholesterol to the phospholipid to the polyethylene glycol modified lipid molecule may be 50:38.5:10:1.5.

For example, the composition may contain the mRNA and the lipid nanoparticles, wherein the mRNA encodes an amino acid sequence shown in SEQ ID NO: 15, the lipid nanoparticles may include the cationic lipids, the cholesterol, the phospholipid and the polyethylene glycol modified lipid molecule, and a molar ratio of the cationic lipids to the cholesterol to the phospholipid to the polyethylene glycol modified lipid molecule may be 50:38.5:10:1.5.

For example, the composition may contain the mRNA and the lipid nanoparticles, wherein the mRNA encodes the amino acid sequence shown in SEQ ID NO: 15, the lipid nanoparticles may contain the DLin-MC3-DMA, the cholesterol, the DSPC and the PEG2000-DMG, and a molar ratio of the DLin-MC3-DMA to the cholesterol to the DSPC to the PEG2000-DMG may be 50:38.5:10:1.5.

For example, the composition may contain the mRNA and the lipid nanoparticles, wherein the mRNA contains a nucleotide sequence shown in SEQ ID NO: 23, the lipid nanoparticles may contain the cationic lipids, the cholesterol, the phospholipid and the polyethylene glycol modified lipid molecule, and a molar ratio of the cationic lipids to the cholesterol to the phospholipid to the polyethylene glycol modified lipid molecule may be 50:38.5:10:1.5.

For example, the composition may contain the mRNA and the lipid nanoparticles, wherein the mRNA contains the amino acid sequence shown in SEQ ID NO: 23, the lipid nanoparticles may contain the DLin-MC3-DMA, the cholesterol, the DSPC and the PEG2000-DMG, and a molar ratio of the DLin-MC3-DMA to the cholesterol to the DSPC to the PEG2000-DMG may be 50:38.5:10:1.5.

### Preparation method

The present application provides a method for preparing a delivery vector in a composition. For example, multilamellar vesicles (MLVs) may be prepared through existing techniques, such as depositing selected lipids on inner walls of suitable containers or vessels; and dissolving the lipids in a suitable solvent and then evaporating the solvent to retain a thin film on an inner side of a vessel, or conducting spray-drying. A water phase may be added to a rotating vessel, leading to the formation of the MLVs. Then, unilamellar vesicles (ULVs) can be formed by homogenization, ultrasound treatment, or extrusion of multilamellar vesicles. In addition, the unilamellar vesicles can be formed through a detergent removal technology.

In the present application, the composition contains the delivery vector (such as lipid nanoparticles), wherein an mRNA may be associated with a surface of the lipid delivery vector and encapsulated within it. For example, in preparing the composition of the present application, the delivery vector of cationic lipids may be associated with the mRNA through electrostatic interaction.

In certain embodiments, the composition contains diagnostic radioactive nuclides, fluorescent substances, or other substances that are detectable *in vivo* and *in vitro.*

In the present application, an appropriate size of the lipid delivery vector (such as the lipid nanoparticles) is selected considering the size of target cells or tissue and the degree of application of liposome to be prepared. In certain embodiments, an mRNA may be delivered to a specific cell or tissue. For example, in order to target liver cells, the size of the lipid delivery vector (such as the lipid nanoparticles) may be determined, so that its size is smaller than the fenestrated gap of the endothelial lining hepatic sinusoid in the liver, allowing the lipid delivery vector (such as the lipid nanoparticles) to easily penetrate these endothelial fenestrated gaps to reach the target liver cells. The lipid delivery vector (such as the lipid nanoparticles) may have a sufficiently large diameter to limit or significantly avoid distribution within certain cells or tissue. In the present application, the size (e.g. diameter) of the lipid delivery vector (such as the lipid nanoparticles) may be in the range of about 25 to 250 nm, such as less than about 250 nm, 175 nm, 150 nm, 125 nm, 100 nm, 75 nm, 50 nm, 25 nm, or 10 nm. For example, the size (e.g. diameter) of the lipid delivery vector (such as the lipid nanoparticles) may be in the range of about 25 to 250 nm, such as about 50 to 200 nm, about 75 to 175 nm, about 75 to 150 nm, or about 75 to 125 nm.

### Vector, cell and pharmaceutical composition

In another aspect, the present application provides a vector, the vector containing a nucleic acid molecule. For example, the vector may be a viral vector, such as an adenovirus vector, an adeno-associated virus vector, and/or a lentivirus vector.

In another aspect, the present application provides a cell, the cell containing the nucleic acid molecule and/or the vector. In the present application, the cell may be a prokaryocyte, such as *E*. *coli.* In the present application, the cell may be a eukaryocyte, such as a yeast cell, an insect cell, a plant cell and an animal cell. In the present application, the cell may be a mammal cell, such as a mouse cell and a human cell. In the present application, the cell may be a 293T cell or a P12 cell.

In another aspect, the present application provides a method for preparing a recombinant protein, and the method includes culturing the cell under a condition of expressing the recombinant protein.

In another aspect, the present application provides a pharmaceutical composition, and the pharmaceutical composition includes the nucleic acid molecule, the composition, the vector and/or the cell, and optionally a pharmaceutically acceptable carrier.

In another aspect, the present application provides a kit or a drug administration device, and the kit or the drug administration device includes the nucleic acid molecule, the composition, the vector, the cell, and/or the pharmaceutical composition. The nucleic acid molecule, the composition, the vector, the cell and/or the pharmaceutical composition of the present application may be included in a single common container, and they may also be optionally combined with one or more therapeutic agents and optionally prepared together in the kit.

### Therapeutic method

In another aspect, the present application provides a method for relieving, preventing and/or treating central and/or peripheral nervous system diseases. The method includes administrating to a subject in need the nucleic acid molecule, the composition, the vector, the cell, and/or the pharmaceutical composition. For example, the central and/or peripheral nervous system diseases may include neurodegenerative diseases. For example, the central and/or peripheral nervous system diseases may include peripheral neuropathy. For example, the peripheral neuropathy includes toxic peripheral neuropathy, hereditary peripheral neuropathy and/or metabolic peripheral neuropathy. For example, the central and/or peripheral nervous system diseases may be included in certain implementations, and the peripheral neuropathy includes toxic peripheral neuropathy, hereditary peripheral neuropathy and/or metabolic peripheral neuropathy.

In certain implementations, the central and/or peripheral nervous system diseases include diabetic peripheral neuropathy, drug-related peripheral neuropathy, hereditary motor sensory neuropathy, post-traumatic repair of peripheral nerves, traumatic optic neuritis and/or Alzheimer's disease.

In another aspect, the present application provides a use of the nucleic acid molecule, the composition, the vector, the cell and/or the pharmaceutical composition in preparation of a medication which may be used for relieving, preventing and/or treating central and/or peripheral nervous system diseases. For example, the central and/or peripheral nervous system diseases may include neurodegenerative diseases. For example, the central and/or peripheral nervous system diseases may include peripheral neuropathy. For example, the peripheral neuropathy includes toxic peripheral neuropathy, hereditary peripheral neuropathy and/or metabolic peripheral neuropathy. For example, the central and/or peripheral nervous system diseases may be included in certain implementations, and the peripheral neuropathy includes toxic peripheral neuropathy, hereditary peripheral neuropathy and/or metabolic peripheral neuropathy.

In another aspect, the present application provides a method for promoting secretion and/or expression of a nerve growth factor (NGF) protein. The method includes using the nucleic acid molecule, the composition, the vector, the cell, and/or the pharmaceutical composition. According to the invention, the method is a non-therapeutic method. According to the invention, the method is an in-vitro and/or ex-vivo method.

In another aspect, the present application provides a method for promoting proliferation and/or differentiation of nerve cells. The method includes using the nucleic acid molecule, the composition, the vector, the cell, and/or the pharmaceutical composition. According to the invention, the method is a non-therapeutic method. According to the invention, the method is an in-vitro and/or ex-vivo method.

In another aspect useful for understanding the invention, the present application provides a method for repairing injured nerve cells. The method includes using the recombinant protein, the nucleic acid molecule, the composition, the vector, the cell, and/or the pharmaceutical composition. For example, the method may be a non-therapeutic method. For example, the method may be an in-vitro and/or ex-vivo method.

Without being limited by any theory, the examples in the following are only intended to illustrate various technical solutions of the present application, and are not intended to limit the scope of the present invention, which is defined by the appended claims.

### EXAMPLES

### Example 1: Design optimization and synthesis of NGF^{R100W} mRNA

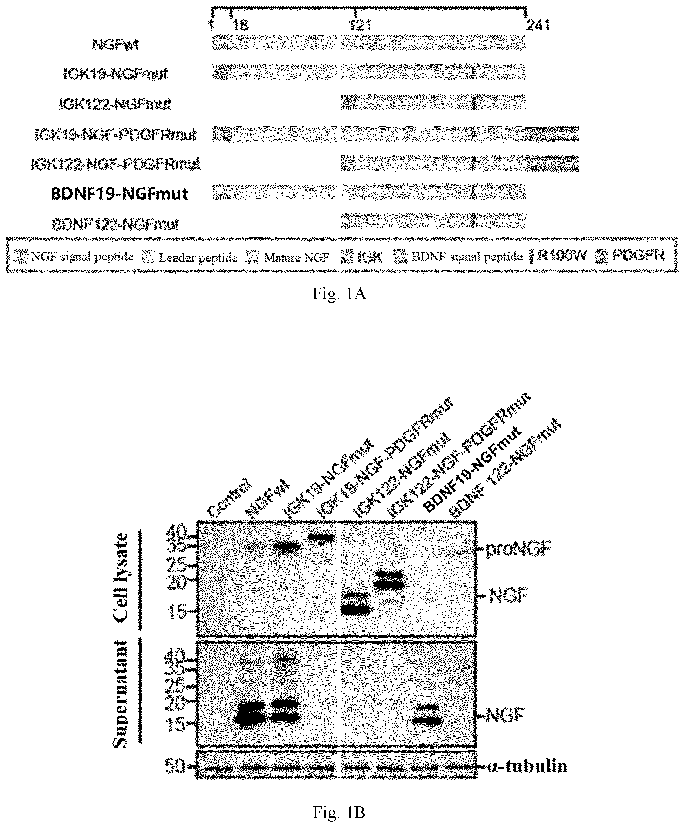

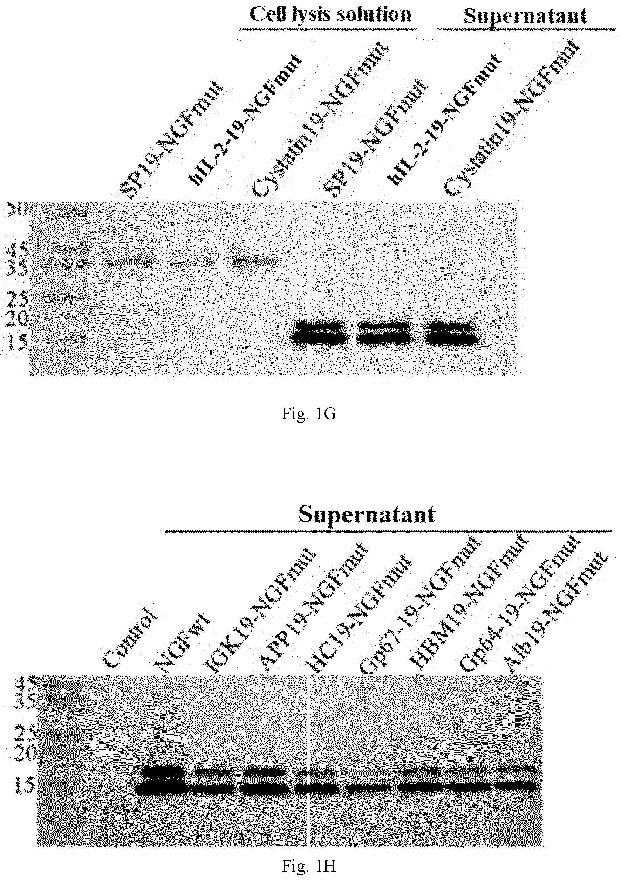

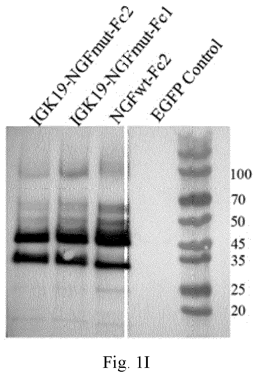

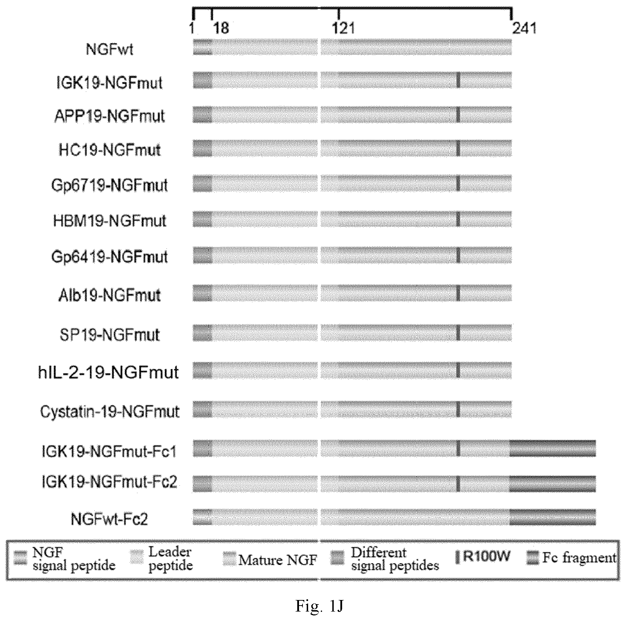

A recombinant protein containing an NGF mutant was constructed by replacing an NGF endogenous signal peptide with an exogenous signal peptide, truncating a leader peptide, and increasing transmembrane anchoring regions, etc. as shown in Fig. 1A or Fig. 1J. NGFwt: a full-length wild type NGF protein, from an N terminal to a C terminal, being NGF signal peptide + leader peptide + mature NGF protein sequentially; NGFmut: a full-length NGF^{R221W} mutant protein, from an N terminal to a C terminal, being NGF signal peptide + leader peptide + mature NGF^{R100W} mutant protein sequentially; IGK19-NGFmut: from an N terminal to a C terminal, being IGK signal peptide + leader peptide + mature NGF^{R100W} mutant protein sequentially; IGK122-NGFmut: from an N terminal to a C terminal, being IGK signal peptide + mature NGF^{R100W} mutant protein sequentially; IGK19-NGF-PDGFRmut: from an N terminal to a C terminal, being IGK signal peptide + leader peptide + mature NGF^{R100W} mutant protein + PDGFR transmembrane anchoring region sequentially; IGK122-NGF-PDGFRmut: from an N terminal to a C terminal, being IGK signal peptide + mature NGF^{R100W} mutant protein + PDGFR transmembrane anchoring region sequentially; BDNF19NGFmut: from an N terminal to a C terminal, being BDNF signal peptide + leader peptide + mature NGF^{R100W} mutant protein sequentially; BDNF122-NGFmut: from an N terminal to a C terminal, being BDNF signal peptide + mature NGF^{R100W} mutant protein sequentially; and amino acid sequences and nucleotide sequences were as shown in Table 1 below.

**Table 1: Sequences of NGF or its mutant recombinant proteins**

| Recombinant protein | Amino acid sequence (SEQ ID NO.) | Coding region sequence (SEQ ID NO.) | Codon-optimized coding region sequence (SEQ ID NO.) | mRNA sequence (SEQ ID NO.) |
|---|---|---|---|---|
| NGFwt | 13 | 9 | 1 | 21 |
| NGFmut | 14 | 10 | 2 | 22 |
| IGK19-NGFmut | 15 | 11 | 3 | 23 |
| IGK122-NGFmut | 16 | - | 4 | 24 |
| IGK19-NGF-PDGFRmut | 17 | - | 5 | 25 |
| IGK122-NGF-PDGFRmut | 18 | - | 6 | 26 |
| BDNF19-NGFmut | 19 | 12 | 7 | 27 |
| BDNF122-NGFmut | 20 | - | 8 | 28 |
| IGK19-NGFmut-Fc1 | 54 | - | 46 | 50 |
| IGK19-NGFmut-Fc2 | 55 | - | 47 | 51 |
| SP-19-NGFmut | 58 | - | - | - |
| hIL-2-19-NGFmut | 59 | - | - | - |
| Cystatin 19-NGFmut | 60 | - | - | - |
| APP-19-NGFmut | 61 | - | - | - |
| HC-19-NGFmut | 62 | - | - | - |
| Gp67-19-NGFmut | 63 | - | - | - |
| HBM-19-NGFmut | 64 | - | - | - |
| Gp64-19-NGFmut | 65 | - | - | - |
| Alb-19-NGFmut | 66 | - | - | - |

The DNA sequence of the above coding region was obtained by in vitro transcription using T7 polymerase. HA tags were added at the 3' terminal and cloned downstream of a T7 promoter on a pSG5L vector. A universal primer for a linear DNA template for in vitro transcription was obtained on the vector, and the DNA template for in vitro transcription was obtained by PCR. The T7 RNA polymerase was used for in vitro transcription, after DNA enzymes were used for digesting the template, *Escherichia coli* poly(A) polymerase EPAP (*E. coli* Poly(A) Polymerase) was used for poly(A) tail addition, and finally an mRNA was obtained through purification recovery.

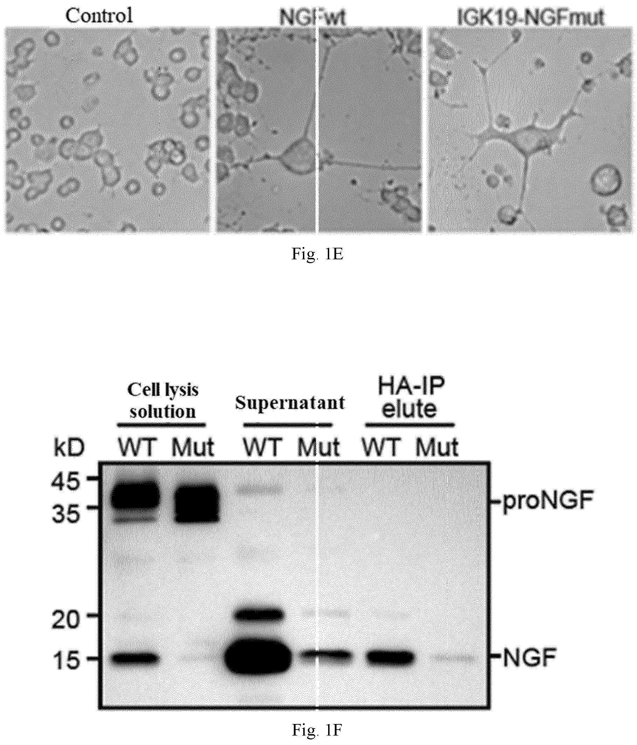

The mRNA was expressed in a 293T tool cell line and Western blot was used to detect an expression amount. The results were shown in Fig. 1B, and protein expression was detected in supernatants of IGK19-NGFmut and BDNF19-NGFmut. Fig. 1F showed that, compared with the wild type NGF protein (WT), the content of secretion of an NGF^{R100W} protein (Mut) without a heterologous signal peptide to a supernatant was relatively low, mostly existing in a cell in the form of a proNGF. In addition, with several other common signal peptides (Fig. 1J), such as APP (azurocidin preproprotein), HC (immunoglobulin heavy chain), SP (signal peptide), Cystatin (Cystatin-S precursor) HBM, Gp64 and Alb (albumin), as shown in Figs. 1G to 1I, the results showed that they all could promote excretion of the NGFmut to a certain extent.

### Example 2: Detection of expression amount of mRNA-modified proteins

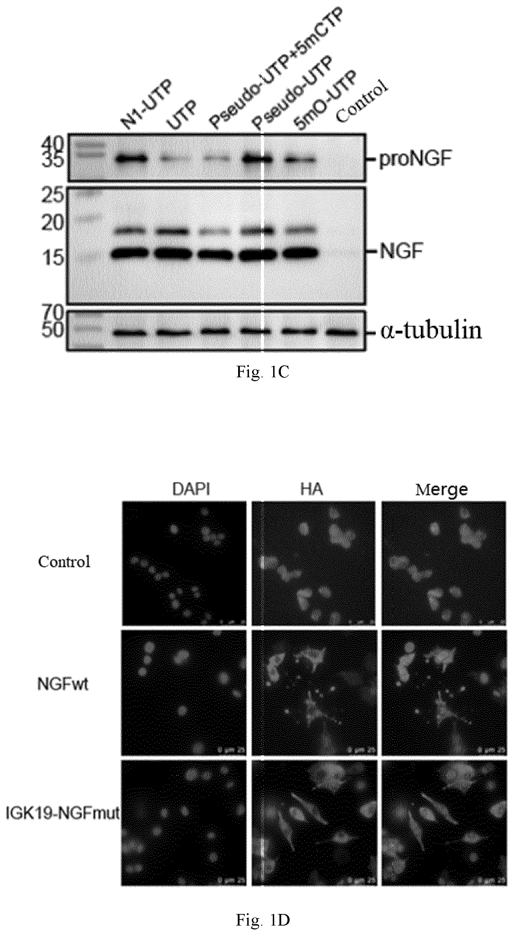

In this example, an IGK19-NGFmut mRNA was modified, that is, nucleotides carrying different chemical modifications were added respectively during transcription to obtain mRNAs carrying chemical modifications, where the introduction of chemical modification nucleotides was 100% replacement. The chemical modifications used were N1-methylpseudouridine-5'-triphosphate (N1-Methylpseudo-UTP, namely N1-UTP), pseudouridine-5'-triphosphate (pseudo-UTP), 5-methoxyuridine-5'-triphosphate (5-Methoxy-UTP, namely 5mO-UTP) and 5-methylcytidine-5'-triphosphate (5-Methyl-CTP, 5mCTP) respectively. The results were shown in Fig. 1C, a protein expression amount obtained from the translation of the IGK19-NGFmut mRNA with different modifications was equivalent, and the protein expression amount with the N1-UTP modification was the highest. The mRNA for subsequent biochemical and functional studies was modified with the N1-UTP.

### Example 3: Detection of function of promoting nerve cell differentiation of NGF^{R100W}

A PC12 cell line was derived from transplantable mouse pheochromocytoma, and the cell had a reversible neuronal phenotype response to an NGF. When PC12 cells were exposed to an environment containing NGF proteins, they could respond within a week, which mainly stopped cell division and extended neural processes. The PC12 cells were cultured in an RPMI1640 medium containing 5% fetal bovine serum and 5% horse serum. When the cells reached the logarithmic growth phase, NGFwt or IGK19-NGFmut mRNA was used for transfection. After 24 hours, the cells were stained with DAPI and HA, and the morphology of the cells was observed under a fluorescence microscope and an electron microscope. As shown in Figs. 1D and 1E, the results showed that the mRNA of IGK19NGFmut, like NGFwt, could be translated and expressed as an NGF^{R100W} mutant protein, and promote the differentiation of the PC12 cells.

### Example 4: Obtaining lipid nanoparticles by encapsulating NGF mRNA with lipids

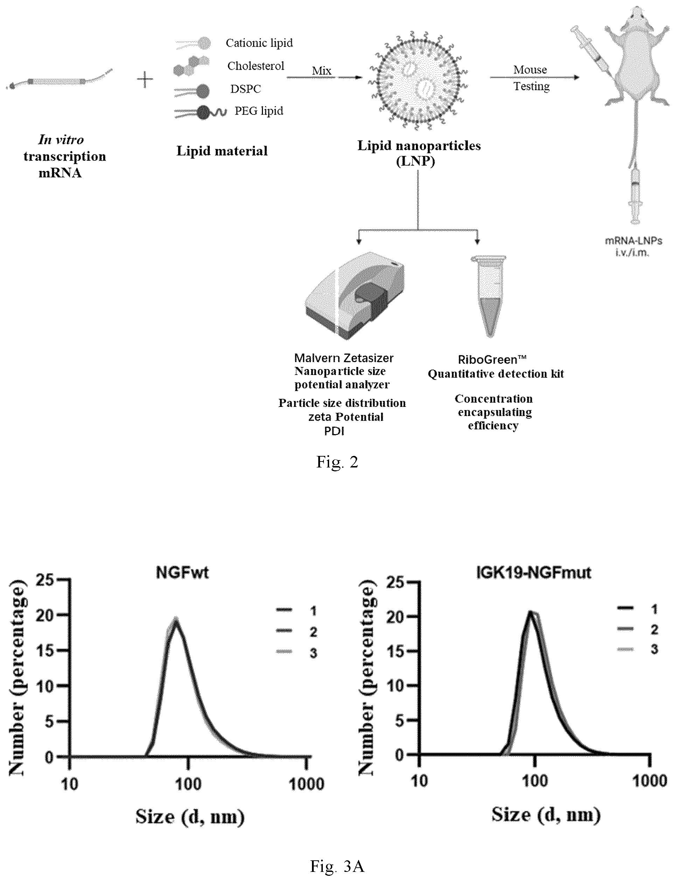

An NGF mRNA and lipid mixture were encapsulated into a lipid nanoparticle (LNP) system to deliver an mRNA, of which the process was as shown in Fig. 2. Four types of lipid molecules were used: cationic lipids (DLin-MC3-DMA), cholesterol, distearyl phosphatidylcholine (DSPC), and polyethylene glycol modified lipid molecules (PEG2000-DMG). The four types of lipids were dissolved in ethanol at a molar ratio of 50:38.5:10:1.5. The lipids dissolved in the ethanol were quickly mixed evenly with an mRNA transcribed in vitro through a T tube, and lipid nanoparticles with a diameter of about 100 nm were obtained by self-packaging. The NGFwt and IGK19-NGFmut transcribed in vitro were mixed and packaged with lipids in a ratio of mRNA: cationic lipids=1:3 (molar ratio) to form nanoparticles LNP. A particle size of the LNPs was detected using a Malvern Zetasizer nanoparticle size potential analyzer, and the results showed that the size of the NGFwt and IGK19-NGFmut were about 100 nm, as shown in Fig. 3A.

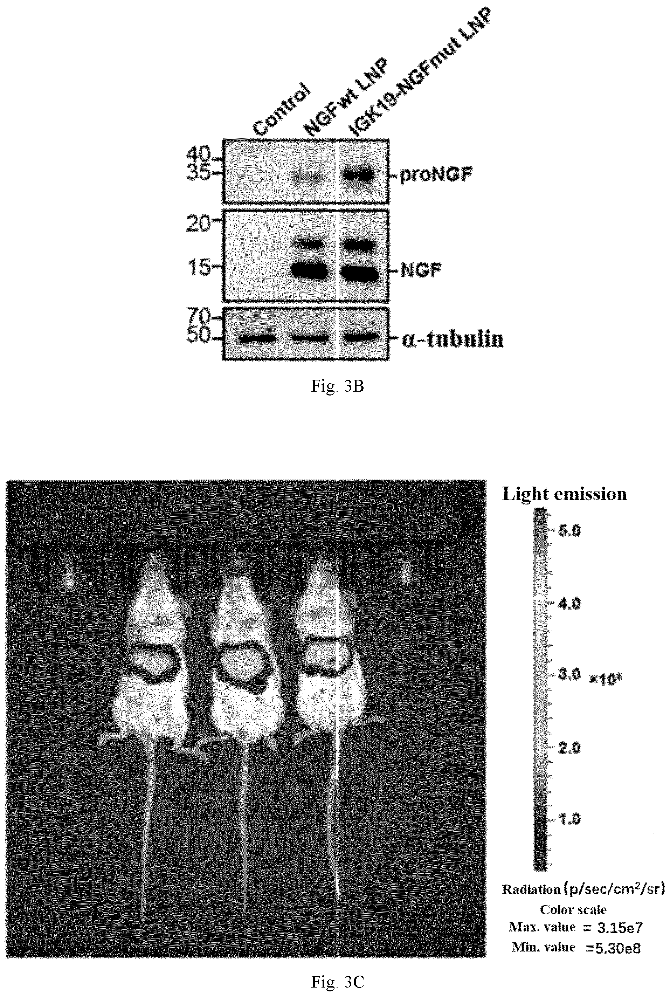

2 µg of NGF LNPs were directly dropwise added to 293T cells for cell transfection, and the expression of mature NGF proteins excreted into a supernatant could be detected in the cells and the supernatant, as shown in Fig. 3B. A fluorescence reporting system in which luciferase oxidized D-luciferin was utilized to emit fluorescence, combined with the biological in vivo fluorescence imaging technology, to determine the localization and expression kinetics of expression of nanoparticles in mice. The mice were injected with nanoparticles encapsulating luciferase mRNAs through the tail vein, and then intraperitoneally injected with luciferin substrates at different time points, and the intensity and localization of biological light generated by oxidation of the substrates by the luciferase were detected by using small animal in vivo imaging. The results showed that the expression efficiency of the nanoparticles was higher when administered through a circulatory pathway, and it was mainly concentrated in the liver, as shown in Fig. 3C.

### Example 5: Validation of low pain-inducing effect of IGK19-NGFmut LNPs in mice

Eight 8-week-old C57BL/6 male mice were divided into two groups, with four mice in each group, and by using plantar injection, each mouse was injected with 2 µg of IGK19-NGFmut LNPs and 2 µg of NGFwt LNPs in plantar foot pad areas of the hind paws of the mice. Mouse behavioral experiments were conducted 8 hours after injection to detect a pain threshold of heat pain in the soles of the mice. The mice were placed on a transparent rack 8 hours after plantar injection, the two groups of mice were given the same intensity of infrared heat source stimulation, and the latent time of paw lifting of the mice was recorded.

Ten 8-week-old C57BL/6 male mice were divided into two groups, with 5 mice in each group, and were injected with 2 µg of IGK19-NGFmut LNPs and 2 µg of NGFwt LNPs in the same way. The mice were placed on a grid frame 8 hours after plantar injection, mechanical stress of different magnitudes was applied to the soles of the mice using von frey filaments, and stress values that could cause the mice to lift their claws were recorded.

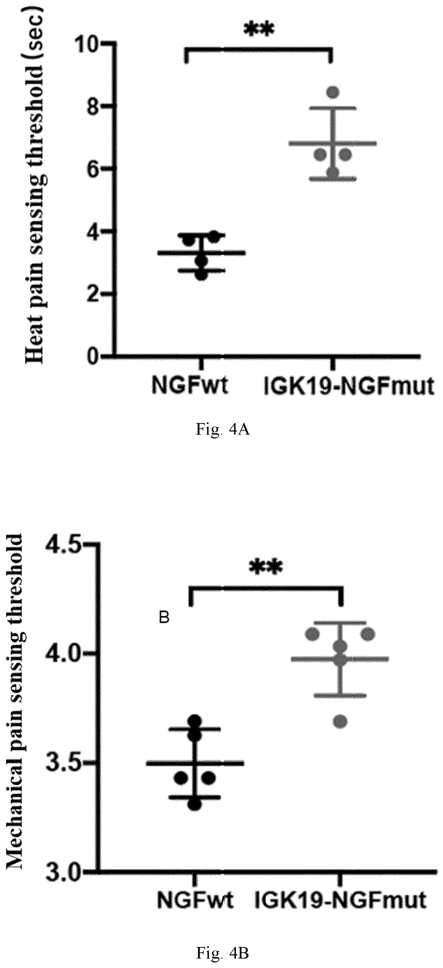

The results showed that the mice injected with the IGK19-NGFmut LNPs had a significantly higher sensitivity threshold to heat pain compared to the control group mice injected with the NGFwt LNPs, as shown in Fig. 4A; and meanwhile, Fig. 4B also showed that the expression of the IGK19-NGFmut mRNAs made the mice less sensitive in the response of mechanical pain compared to the expression of the NGFwt, indicating that the IGK19-NGFmut LNPs had a lower pain-inducing effect on mice compared to the expression of the NGFwt LNPs in the soles of the mice.

### Example 6: Validation of nerve repair effect of IGK19-NGFmut LNPs in mice with peripheral nerve injuries

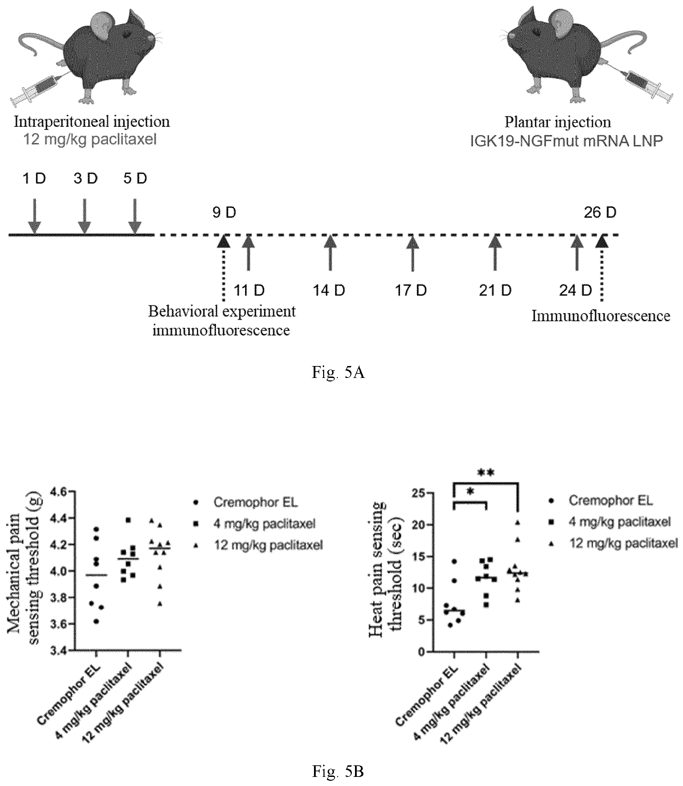

Modeling of mouse peripheral nerve injuries was conducted with fifteen 8-week-old C57BL/6 male mice by using a method of intraperitoneal injection of paclitaxel every other day. One week after the completion of modeling, the mice were divided into three groups, with 6 mice in each group injected with IGK19-NGFmut LNPs and NGFwt LNPs, and only 3 mice in a modeling group. By using plantar injection, each mouse was injected with 2 µg of the IGK19-NGFmut LNPs and 2 µg of the NGFwt LNPs in the plantar foot pad area of the hind paws of the mice, and the injection was conducted every three days. After 5 injections, the pain threshold of heat pain and the pain threshold of mechanical pain in the soles of the mice were tested. The mice were placed on a grid frame, mechanical stress of different magnitudes was applied to the soles of the mice using von frey filaments, and stress values that could cause the mice to lift their claws were recorded. After completing the mechanical pain threshold test, a 2-hour buffer period was given, then the two groups of mice were placed on a hot plate at 55°C, and the latent time of paw lifting of the mice was recorded. The experimental process was as shown in Fig. 5A.

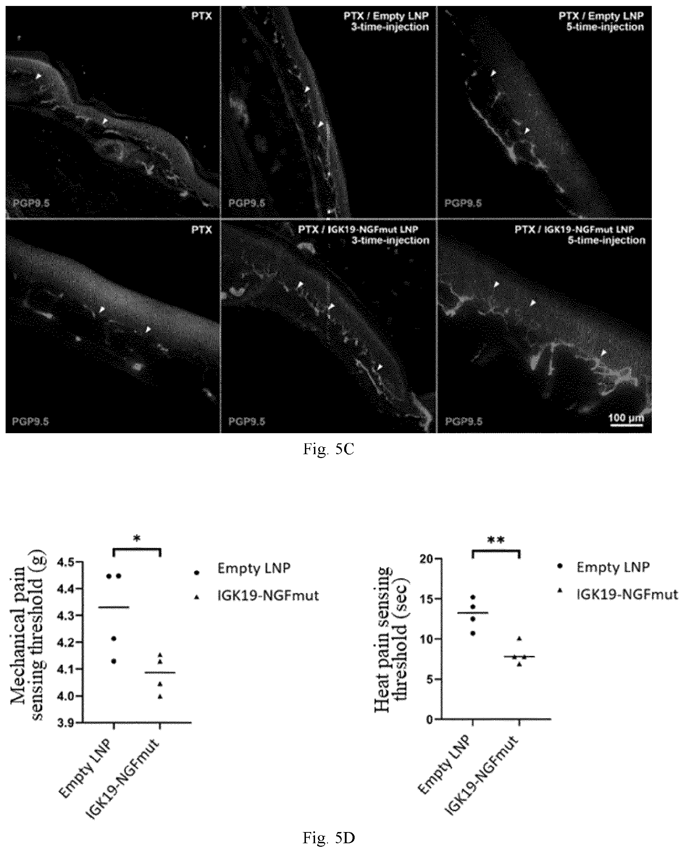

The results of heat pain and mechanical pain in Fig. 5B showed that the mouse peripheral nerve injuries caused by the injection dose of 12 mg/kg of paclitaxel could all cause an increase in plantar pain threshold of the mice. As the number of injections increased, the continuity and density of peripheral nerves of the mice injected with the IGK19-NGFmut LNPs could be increased obviously compared to the control group mice injected with the NGFwt LNPs, as shown in Fig. 5C; and meanwhile, Fig. 5D also showed that the expression of the IGK19-NGFmut mRNAs made the mice more sensitive in the response of mechanical pain and heat pain compared to the expression of NGFwt, indicating that the expression of the IGK19-NGFmut LNPs in the soles of the mice could repair peripheral nerve injuries caused by paclitaxel.

The above detailed explanation is provided in the form of explanation and examples, and is not intended to limit the scope of the attached claims.

## Claims

1. An isolated nucleic acid molecule encoding a recombinant protein as shown in SEQ ID NO: 15,
wherein the nucleic acid molecule is an RNA,
wherein the RNA contains modifications at one or more positions selected from: a 5' cap, a 5' untranslated region, an open reading frame, a 3 'untranslated region, and a poly A tail, wherein the RNA contains at least one modified nucleotide,
wherein the modified nucleotide contained in the nucleic acid molecule contains pseudouridine-triphosphate (pseudo-UTP), and
wherein the nucleotide sequence of the RNA is shown in SEQ ID NO: 23.

2. A composition, comprising: (a) an mRNA with a nucleotide sequence shown in SEQ ID NO: 23, and (b) a delivery vector;
wherein the mRNA contains modifications at one or more positions selected from: a 5' cap, a 5' untranslated region, an open reading frame, a 3 'untranslated region, and a poly A tail; and
wherein the mRNA contains at least one modified nucleotide, and wherein the modified nucleotide is pseudouridine-triphosphate (pseudo-UTP).

3. The composition according to claim 2, wherein the delivery vector comprises DLin-MC3-DMA, cholesterol, distearoylphosphatidylcholine (DSPC) and PEG2000-DMG, and wherein a molar ratio of the DLin-MC3-DMA to the cholesterol to the DSPC to the PEG2000-DMG is 50 : 38.5 : 10 : 1.5.

4. The composition according to claim 2, wherein the delivery vector has a diameter of 80 nm to 200 nm.

5. The composition according to claim 2, wherein the mRNA is encapsulated in the delivery vector.

6. A vector, comprising the nucleic acid molecule according to claim 1.

7. A cell, comprising the nucleic acid molecule according to claim 1, and/or the vector according to claim 6.

8. A method for preparing a recombinant protein, comprising culturing the cell according to claim 7 under a condition of expressing a recombinant protein as shown in SEQ ID NO: 15.

9. A pharmaceutical composition, comprising the nucleic acid molecule according to claim 1, the composition according to any one of claims 2-5, the vector according to claim 6, and/or the cell according to claim 7, and optionally a pharmaceutically acceptable carrier.

10. A kit or a drug delivery device, comprising the nucleic acid molecule according to claim 1, the composition according to any one of claims 2-5, the vector according to claim 6, the cell according to claim 7, and/or the pharmaceutical composition according to claim 9.

11. The nucleic acid molecule according to claim 1, the composition according to any one of claims 2-5, the vector according to claim 6, the cell according to claim 7, and/or the pharmaceutical composition according to claim 9, for use in relieving, preventing and/or treating central and/or peripheral nervous system diseases.

12. A method for promoting secretion and/or expression of a nerve growth factor (NGF) protein, comprising using the nucleic acid molecule according to claim 1, the composition according to any one of claims 2-5, the vector according to claim 6, the cell according to claim 7, and/or the pharmaceutical composition according to claim 9;
wherein the method is a non-therapeutic method; and
wherein the method is an *in vitro* and/or *ex vivo* method.

13. A method for promoting proliferation and/or differentiation of neural cells, comprising using the nucleic acid molecule according to claim 1, the composition according to any one of claims 2-5, the vector according to claim 6, the cell according to claim 7, and/or the pharmaceutical composition according to claim 9;
wherein the method is a non-therapeutic method; and
wherein the method is an *in vitro* and/or *ex vivo* method.

## Patentansprüche

1. Isoliertes Nukleinsäuremolekül, das ein rekombinantes Protein, wie in SEQ ID NO: 15 gezeigt, kodiert,
wobei das Nukleinsäuremolekül eine RNA ist,
wobei die RNA Modifikationen an einer oder mehreren Positionen enthält, die ausgewählt sind aus: einer 5'-Kappe, einer 5'-untranslatierten Region, einem offenen Leserahmen, einer 3'-untranslatierten Region und einem Poly-A-Schwanz,
wobei die RNA mindestens ein modifiziertes Nukleotid enthält,
wobei das in dem Nukleinsäuremolekül enthaltene modifizierte Nukleotid Pseudouridintriphosphat (Pseudo-UTP) enthält, und
wobei die Nukleotidsequenz der RNA in SEQ ID NO: 23 gezeigt ist.

2. Zusammensetzung, umfassend: (a) eine mRNA mit einer Nukleotidsequenz, die in SEQ ID NO: 23 gezeigt ist, und (b) einen Abgabevektor;
wobei die mRNA Modifikationen an einer oder mehreren Positionen enthält, ausgewählt aus: einer 5'-Kappe, einer 5'-untranslatierten Region, einem offenen Leserahmen, einer 3'-untranslatierten Region und einem Poly-A-Schwanz; und
wobei die mRNA mindestens ein modifiziertes Nukleotid enthält, und wobei das modifizierte Nukleotid Pseudouridintriphosphat (Pseudo-UTP) ist.

3. Zusammensetzung nach Anspruch 2, wobei der Abgabevektor DLin-MC3-DMA, Cholesterin, Distearoylphosphatidylcholin (DSPC) und PEG2000-DMG umfasst, und wobei ein Molverhältnis von DLin-MC3-DMA zu Cholesterin zu DSPC zu PEG2000-DMG 50 : 38,5 : 10 : 1,5 beträgt.

4. Zusammensetzung nach Anspruch 2, wobei der Abgabevektor einen Durchmesser von 80 nm bis 200 nm aufweist.

5. Zusammensetzung nach Anspruch 2, wobei die mRNA in dem Abgabevektor eingekapselt ist.

6. Vektor, umfassend das Nukleinsäuremolekül nach Anspruch 1.

7. Zelle, umfassend das Nukleinsäuremolekül nach Anspruch 1 und/oder den Vektor nach Anspruch 6.

8. Verfahren zur Herstellung eines rekombinanten Proteins, umfassend das Kultivieren der Zelle nach Anspruch 7 unter einer Bedingung, unter der ein rekombinantes Protein wie in SEQ ID NO:15 gezeigt exprimiert wird.

9. Pharmazeutische Zusammensetzung, umfassend das Nukleinsäuremolekül nach Anspruch 1, die Zusammensetzung nach einem der Ansprüche 2-5, den Vektor nach Anspruch 6 und/oder die Zelle nach Anspruch 7, und optional einen pharmazeutisch annehmbaren Träger.

10. Kit oder Arzneimittelabgabevorrichtung, umfassend das Nukleinsäuremolekül nach Anspruch 1, die Zusammensetzung nach einem der Ansprüche 2-5, den Vektor nach Anspruch 6, die Zelle nach Anspruch 7 und/oder die pharmazeutische Zusammensetzung nach Anspruch 9.

11. Nukleinsäuremolekül nach Anspruch 1, Zusammensetzung nach einem der Ansprüche 2-5, Vektor nach Anspruch 6, Zelle nach Anspruch 7 und/oder pharmazeutische Zusammensetzung nach Anspruch 9 zur Anwendung bei der Linderung, Vorbeugung und/oder Behandlung von Erkrankungen des zentralen und/oder peripheren Nervensystems.

12. Verfahren zur Förderung der Sekretion und/oder Expression eines Nervenwachstumsfaktor (NGF)-Proteins, umfassend die Verwendung des Nukleinsäuremoleküls nach Anspruch 1, der Zusammensetzung nach einem der Ansprüche 2-5, des Vektors nach Anspruch 6, der Zelle nach Anspruch 7 und/oder der pharmazeutischen Zusammensetzung nach Anspruch 9;
wobei das Verfahren ein nicht-therapeutisches Verfahren ist; und
wobei das Verfahren ein *in vitro-* und/oder *ex vivo*-Verfahren ist.

13. Verfahren zur Förderung der Proliferation und/oder Differenzierung von Nervenzellen, umfassend die Verwendung des Nukleinsäuremoleküls nach Anspruch 1, der Zusammensetzung nach einem der Ansprüche 2-5, des Vektors nach Anspruch 6, der Zelle nach Anspruch 7 und/oder der pharmazeutischen Zusammensetzung nach Anspruch 9;
wobei das Verfahren ein nicht-therapeutisches Verfahren ist; und
wobei das Verfahren ein *in vitro-* und/oder *ex vivo*-Verfahren ist.

## Revendications

1. Molécule d'acide nucléique isolée codant pour une protéine recombinante telle que représentée dans SEQ ID NO : 15,
où la molécule d'acide nucléique est un ARN,
où l'ARN contient des modifications à une ou plusieurs positions choisies parmi : une coiffe 5', une région 5' non traduite, un cadre de lecture ouvert, une région 3' non traduite et une queue poly A,
où l'ARN contient au moins un nucléotide modifié,
où le nucléotide modifié contenu dans la molécule d'acide nucléique contient de la pseudouridine-triphosphate (pseudo-UTP), et
où la séquence nucléotidique de l'ARN est représentée dans SEQ ID NO : 23.

2. Composition comprenant : (a) un ARNm avec une séquence nucléotidique représentée dans SEQ ID NO : 23, et (b) un vecteur de délivrance ;
où l'ARNm contient des modifications à une ou plusieurs positions choisies parmi : une coiffe 5', une région 5' non traduite, un cadre de lecture ouvert, une région 3' non traduite et une queue poly A ; et
où l'ARNm contient au moins un nucléotide modifié, et où le nucléotide modifié est de la pseudouridine-triphosphate (pseudo-UTP)

3. Composition selon la revendication 2, où le vecteur de délivrance comprend du DLin-MC3-DMA, du cholestérol, de la distéaroylphosphatidylcholine (DSPC) et du PEG2000-DMG, et où le rapport molaire entre le DLin-MC3-DMA, le cholestérol, la DSPC et le PEG2000-DMG est de 50 : 38,5 : 10 : 1,5.

4. Composition selon la revendication 2, où le vecteur de délivrance a un diamètre de 80 nm à 200 nm.

5. Composition selon la revendication 2, où l'ARNm est encapsulé dans le vecteur de délivrance.

6. Vecteur comprenant la molécule d'acide nucléique selon la revendication 1.

7. Cellule comprenant la molécule d'acide nucléique selon la revendication 1 et/ou le vecteur selon la revendication 6.

8. Procédé de préparation d'une protéine recombinante, comprenant la culture de la cellule selon la revendication 7 sous une condition d'expression d'une protéine recombinante telle que représentée dans SEQ ID NO : 15.

9. Composition pharmaceutique, comprenant la molécule d'acide nucléique selon la revendication 1, la composition selon l'une quelconque des revendications 2 à 5, le vecteur selon la revendication 6 et/ou la cellule selon la revendication 7, et éventuellement un excipient pharmaceutiquement acceptable.

10. Kit ou dispositif d'administration de médicament, comprenant la molécule d'acide nucléique selon la revendication 1, la composition selon l'une quelconque des revendications 2 à 5, le vecteur selon la revendication 6, la cellule selon la revendication 7 et/ou la composition pharmaceutique selon la revendication 9.

11. Molécule d'acide nucléique selon la revendication 1, composition selon l'une quelconque des revendications 2 à 5, vecteur selon la revendication 6, cellule selon la revendication 7 et/ou composition pharmaceutique selon la revendication 9, pour utilisation pour soulager, prévenir et/ou traiter des maladies du système nerveux central et/ou périphérique.

12. Procédé pour favoriser la sécrétion et/ou l'expression d'une protéine du facteur de croissance nerveuse (NGF), comprenant l'utilisation de la molécule d'acide nucléique selon la revendication 1, de la composition selon l'une quelconque des revendications 2 à 5, du vecteur selon la revendication 6, de la cellule selon la revendication 7 et/ou de la composition pharmaceutique selon la revendication 9 ;
où le procédé est un procédé non thérapeutique ; et
où le procédé est un procédé *in vitro* et/ou *ex vivo.*

13. Procédé pour favoriser la prolifération et/ou la différenciation de cellules neurales, comprenant l'utilisation de la molécule d'acide nucléique selon la revendication 1, de la composition selon l'une quelconque des revendications 2 à 5, du vecteur selon la revendication 6, de la cellule selon la revendication 7 et/ou de la composition pharmaceutique selon la revendication 9 ;
où le procédé est un procédé non thérapeutique ; et
où le procédé est un procédé *in vitro* et/ou *ex vivo.*
